# EUROPEAN PATENT SPECIFICATION

(11) **EP 2 073 733 B1**
(45) Date of publication and mention of the grant of the patent: **30.12.2015**
(21) Application number: 07825065.1
(22) Date of filing: 05.09.2007
(51) Int. Cl.: A61B 17/70

(54) **INTERSPINOUS SPINAL PROSTHESIS**
INTERSPINÖSE WIRBELSÄULENPROTHESE
PROTHÈSE VERTÉBRALE INTERÉPINEUSE

(30) Priority: 07.09.2006 US 842652 P; 20.10.2006 FR 0609209
(43) Date of publication of application: 01.07.2009
(73) Proprietor: Kyphon SÀRL, 2000 Neuchâtel (CH)
(72) Inventor: Taylor, Jean, 06400 Cannes (FR)
(74) Representative: Price, Nigel John King
(86) International application number: PCT/IB2007/002566
(87) International publication number: WO 2008/029260

(56) References cited:
- WO-A-03/103519
- WO-A-2005/110258
- WO-A-2006/086241
- FR-A- 2 730 156
- US-A- 5 011 484
- US-A1- 2004 243 239

## Description

The present invention relates to an interspinous spinal prosthesis.

### STATE OF THE ART

Interspinous spinal prostheses have been developed as an alternative to interpedicular osteosynthesis devices. The interspinous gap, thus maintained, provides a certain degree of stability by reducing the play between the posterior rims of the vertebrae.

It is essential for this type of implant to conform to the morphology of the spinous processes to prevent migration or forward or rear sliding. This requirement is, however, problematic between the fifth lumbar vertebra (L5) and the first sacral vertebra (S1) because the S1 spinous process very often protrudes very little (agenesis) and therefore cannot constitute a stable bearing point.

The solutions imagined to date have tried to use bearing on the sacrum, either through hooks incorporated in the device and bearing on the upper edge of the sacrum, or using side attachments or transverse links fastened by screws going through the ala sacralis. Due to the specific biomechanics of the lumbosacral joint, all of these solutions are both delicate to implant and unsatisfactory from a mechanical standpoint. However, degenerative pathologies affect several consecutive vertebral joints or evolve such that they affect several consecutive vertebral joints. It is therefore essential to be able to extend the treatment method from a given vertebra toward the joint located above and especially the joint located below, namely L5 - S1 involving an L4 - L5 treatment.

Documents FR 2 623 085, WO 2005/110258, WO 2006/086241 and FR 2 828 398 describe various types of interspinous implants, but which do not provide a satisfactory solution to the aforementioned problem of providing an implant for which migration or forward or rear sliding is prevented, which remaining relatively easy to implant and providing satisfaction from a mechanical standpoint.

US 2004/0243239 discloses a spinal prosthesis according to the precharacterising portion of claim 1.

### SUMMARY OF THE INVENTION

To resolve the abovementioned problem, a prosthesis according to claim 1 is provided.

The invention was designed based on the two following observations, relative to the L5 - S1 stage: on one hand, the supraspinous ligamentary continuity ends at the L5 vertebra, then irradiates; on the other hand, the axis of the spinal cord forms a closed angle, meaning acute, with the lower edge of the spinous process of said first vertebra L5. As a result, preserving the supraspinous ligamentary continuity is not imperative at this level, whereas the support provided by the spinal process of said first vertebra L5 is anatomically weak. During an extension of the vertebral column, the lower edge of the spinal process of the L5 vertebra will come into contact, and, if necessary, bear against the spur formed by the spinal process of said second vertebra, namely the S1 vertebra in this case, which is triangular in shape. The implant according to the invention, slid under this spinal process, will be stuck in the interspinous space while maintaining a gap favorable to soothing the painful newly formed interspinous and inter-joint contacts, while also stretching the capsuloligamentary elements.

In a first embodiment, the implant is distinct and separate from an interspinous implant intended to be placed at the vertebral joint situated above said first vertebra (generally L5), namely between said first vertebra (L5) and a third vertebra (L4). In this case, the implant is U-shaped and is provided with a link at its edge or going through it. The implant is forcibly slid into the interspinous space of the L5 and S1 vertebrae and fits under the lower edge of the L5 spinous process, against which it bears. The link keeps the implant in place by squeezing.

In a second embodiment, the implant is integral with a shock absorbing upper interspinous part intended to be provided on the vertebral joint between said first vertebra (L5) and a third vertebra (L4) above first vertebra (L5); the implant defines, with this upper interspinous part, an opening allowing close, even more or less forced, insertion of the implant on the spinous process of said first vertebra (generally L5). In this case, the link goes through said upper interspinous part to allow the joining of its two side strands.

The implant according to the invention can be "for extension", meaning lengthening, having the effect of increased maintaining of the interspinous device (which is separate from the implant in the first embodiment) or, in said second embodiment, of said upper interspinous part, by preventing forward or rear sliding of its intermediate part.

The implant according to the invention, thanks to its presence on the underlying vertebral joint (generally L5 - S1) and its perfect fixing to the spinous process of said first vertebra (L5), solves the problem of sliding of an interspinous prosthesis being provided on the vertebral joint between said first and third vertebrae (generally L4 - L5) relative to the upper edge of the spinous process of said first vertebra (L5), this risk of sliding resulting from the tilt of this upper edge.

All of the preceding remarks may be transposed from the lumbar area to the cervical and dorsal area.

The implant is preferably made of a viscoelastic material, particularly with a fairly high gradient, optimizing both the stability of the implant by a self-tightening effect around the spinous process and providing a shock absorbing effect for the spinous processes of the first and second vertebrae, generally L5 and S1, respectively. Thus, one can compensate for agenesis of the spinous process of said second vertebra (S 1) given that the bearing is previously moved to the posterior junction of the two half-laminae of the sacrum.

The elasticity module of this malleable viscoelastic shock absorbing material is advantageously less than that of a cortical bone so as to avoid bone weakening.

The outer covering may be in a woven textile material or in any other material with a suitable canvas.

Said intermediate portion of the implant may comprise at least one recess or notch intended to allow precise application of the implant against the lower edge of the spinous process in said first vertebra (generally L5) and/or against the second vertebra (generally S1), and therefore allowing stabilization of this implant relative to that or these spinous process(es).

Preferably, the free end of at least one of the side portions of the implant comprises a recess or a notch enabling the implant to interlock with an interspinous implant intended to be placed between said first and third vertebrae. Preferably, each of the free ends of the two side portions of the implant comprises a recess or notch of this type.

Moreover, the materials required for each segment of this assembly thus formed may differ, thereby forming a composite assembly with variable stiffness.

### BRIEF DESCRIPTION OF THE FIGURES

The figures illustrate two embodiments of the invention.
Figure 1 is a back view, after implantation, according to a first embodiment;
figure 2 is a view similar to figure 1, according to a variation of the embodiment, and
figure 3 is a back view, after implantation, according to a second embodiment.

### DISCUSSION OF PREFERRED EMBODIMENTS

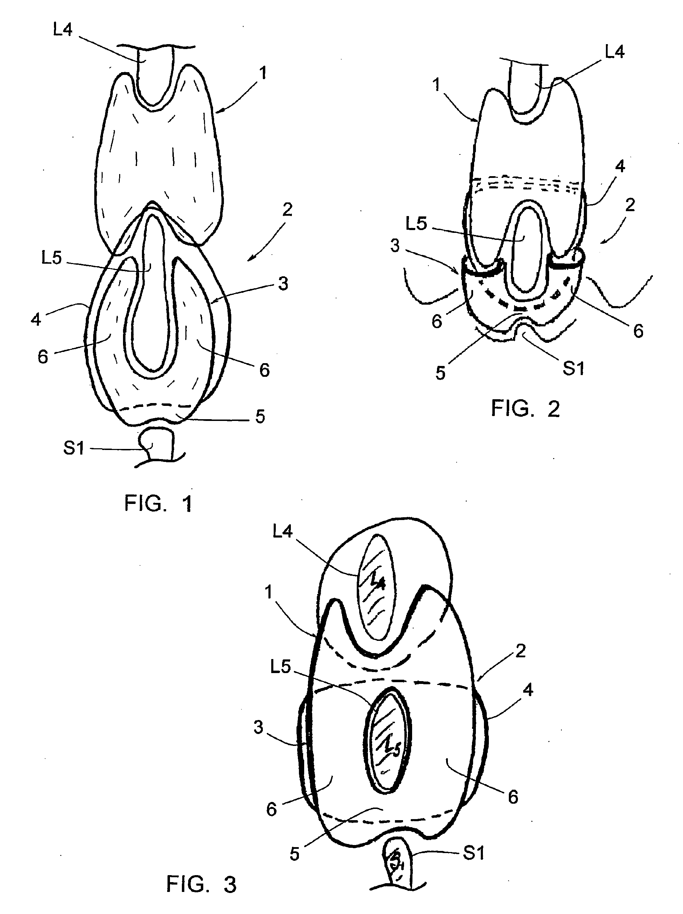

Figure 1, annexed, shows three successive spinous processes, generally L4, L5 and S1. Between the L4 and L5 spinous processes is placed an interspinous implant 1, in particular that known under the name "DIAM".

The prosthesis 2 according to the invention comprises an implant 3 and a link 4.

The implant 3 has a U-shape, meaning it comprises an intermediate portion 5 and two side portions 6. At least the intermediate portion 5 is shock absorbing. This implant 3 is intended to envelope the spinous process of L5, said intermediate portion 5 being intended to be engaged between this spinous process and the S1 spinous process.

The link 4 goes through said intermediate portion 5 and forms two side strands which can go alongside said side portions 6 and be connected to each other so as to closely stick said intermediate portion 5 against the lower edge of the L5 spinous process.

Figure 2 shows a variation of this embodiment wherein the free ends of the side portions 6 comprise recesses enabling them to interlock on the free ends of the lower protruding side portions comprised by the interspinous implant 1. The link 4 is then engaged through the central part of the implant 1.

In a second embodiment, shown in figure 3, the implant 3 is integral with an upper shock absorbing interspinous part 1 comparable to the interspinous implant 1 of the preceding version. The implant 3 defines, with this upper interspinous part 1, an opening allowing close insertion on the spinous process of the L5 vertebra. In this case, the link 4 goes through said upper interspinous part 1 to enable the connection of its two side strands.

## Claims

1. Spinal prosthesis (2), comprising:
- a U-shaped implant (3), comprising an intermediate portion (5) and two side portions (6), at least the intermediate portion (5) being shock absorbing; this implant (3) is intended to envelope the spinous process of a first vertebra (L5), said intermediate portion (5) being intended to be engaged between this spinous process and an area of a second vertebra (S1), underlying; and
- a link (4) crossing through the intermediate portion (5) and forming two side strands which are able to go alongside or cross said side portions (6) and to be connected to each other so as to closely stick said intermediate portion (5) against the lower edge of the spinous process of said first vertebra (L5);
wherein the implant (3) comprises a core and an outer covering;
**characterized in that**:
the core is a composite structure, comprising an inner element made of a non-compressible material and an outer element covering the inner element, forming a layer thicker than the inner element in a malleable viscoelastic shock absorbing material.

2. Spinal prosthesis (2) according to claim 1, further comprisingan interspinous implant (1) intended to be placed at the vertebral joint situated above said first vertebra (L5), namely between said first vertebra (L5) and a third vertebra (L4), and wherein the implant (3) is distinct and separate from said interspinous implant (1).

3. Spinal prosthesis (2) according to claim 1, further comprising a shock absorbing upper interspinous part (1) intended to be provided on the vertebral joint between said first vertebra (L5) and a third vertebra (L4) above this first vertebra (L5); wherein the implant (3) is integral with the upper interspinous part (1), and the implant (3) defines, with this upper interspinous part (1), an opening allowing insertion of the implant (3) on the spinous process of said first vertebra (L5).

4. Spinal prosthesis (2) according to any of claims 1 to 3, **characterized in that** the outer covering is in a woven textile material or in any other material with a suitable canvas.

5. Spinal prosthesis (2) according to any of claims 1 to 4, **characterized in that** said intermediate portion (5) of the implant (3) comprises at least one recess or notch intended to allow precise application of the implant against the lower edge of the spinous process of said first vertebra (L5) and/or against said second vertebra (S1), and therefore allowing stabilization of this implant relative to that or these spinous process(es).

6. Spinal prosthesis (2) according to any of claims 2 to 4, **characterized in that** the free end of at least one of the side portions (6) of the implant (3) comprises a recess or a notch enabling the implant (3) to interlock with an interspinous implant (1) intended to be placed between said first and third vertebrae (L4, L5).

## Patentansprüche

1. Spinalprothese (2), umfassend:
- ein U-förmiges Implantat (3), welches einen Zwischenabschnitt (5) und zwei Seitenabschnitte (6) umfasst, wobei zumindest der Zwischenabschnitt (5) stoßabsorbierend ist; wobei dieses Implantat (3) ausgelegt ist, um den Dornfortsatz einer ersten Bandscheibe (L5) zu umhüllen, wobei der Zwischenabschnitt (5) ausgelegt ist, um zwischen diesem Dornfortsatz und einem Bereich einer zweiten darunterliegenden Bandscheibe (S1) in Eingriff zu kommen;
und
- eine Verbindung (4), welche den Zwischenabschnitt (5) durchquert und zwei Seitenstränge ausbildet, die in der Lage sind, längsseits oder durch die Seitenabschnitte (6) zu verlaufen und miteinander verbunden zu werden, um den Zwischenabschnitt (5) dicht gegen die untere Kante des Dornfortsatzes der ersten Bandscheibe (L5) zu klemmen;
wobei das Implantat (3) einen Kern und eine Außenabdeckung umfasst;
**dadurch gekennzeichnet, dass**:
der Kern eine Verbundstoff-Struktur ist, welche ein inneres Element, das aus einem nicht zusammendrückbaren Material hergestellt ist, und ein äußeres Element, welches das innere Element überdeckt und eine Schicht in einem dehnbaren viskoelastischen stoßabsorbierenden Material ausbildet, welche dicker als das innere Element ist, umfasst.

2. Spinalprothese (2) nach Anspruch 1, darüber hinaus ein interspinales Implantat (1) umfassend, welches ausgelegt ist, um an der Wirbelverbindung, welche sich oberhalb der ersten Bandscheibe (L5), nämlich zwischen der ersten Bandscheibe (L5) und einer dritten Bandscheibe (L4), befindet, angeordnet zu werden, und wobei sich das Implantat (3) deutlich von dem interspinalen Implantat (1) unterscheidet und getrennt von diesem ist.

3. Spinalprothese (2) nach Anspruch 1, darüber hinaus ein stoßabsorbierendes oberes interspinales Teil (1) umfassend, welches ausgelegt ist, um auf der Wirbelverbindung zwischen der ersten Bandscheibe (L5) und einer dritten Bandscheibe (L4) oberhalb dieser ersten Bandscheibe (L5) bereitgestellt zu sein; wobei das Implantat (3) integral mit dem oberen interspinalen Teil (1) ist, und wobei das Implantat (3) mit diesem oberen interspinalen Teil (1) eine Öffnung definiert, welche eine Einführung des Implantats (3) auf den Dornfortsatz der ersten Bandscheibe (L5) ermöglicht.

4. Spinalprothese (2) nach einem der Ansprüche 1 bis 3, **dadurch gekennzeichnet, dass** die Außenabdeckung in einem gewebten Textilmaterial oder in irgendeinem anderen Material mit einem geeigneten Kanevas ist.

5. Spinalprothese (2) nach einem der Ansprüche 1 bis 4,
**dadurch gekennzeichnet, dass** der Zwischenabschnitt (5) des Implantats (3) zumindest eine Vertiefung oder eine Nut umfasst, welche ausgelegt ist, um ein genaues Aufbringen des Implantats an der unteren Kante des Dornfortsatzes der ersten Bandscheibe (L5) und/oder an der zweiten Bandscheibe (S1) zu ermöglichen und dadurch eine Stabilisierung dieses Implantats relativ zu dem Dornfortsatz oder diesen Dornfortsätzen zu ermöglichen.

6. Spinalprothese (2) nach einem der Ansprüche 2 bis 4,
**dadurch gekennzeichnet, dass** das freie Ende von zumindest einem der Seitenabschnitte (6) des Implantats (3) eine Vertiefung oder eine Nut umfasst, welche dem Implantat ermöglicht, sich mit einem interspinalen Implantat (1) zu verhaken, welches ausgelegt ist, um zwischen der ersten und der dritten Bandscheibe (L4, L5) angeordnet zu sein.

## Revendications

1. Prothèse vertébrale (2), comprenant :
- un implant en forme de U (3), comprenant une partie intermédiaire (5) et deux parties latérales (6), au moins la partie intermédiaire (5) absorbant les chocs ; cet implant (3) étant destiné à envelopper l'apophyse épineuse d'une première vertèbre (L5), ladite partie intermédiaire (5) étant destinée à venir s'engager entre cette apophyse épineuse et une région d'une seconde vertèbre (S1), située au-dessous ; et
- un élément de liaison (4) traversant la partie intermédiaire (5) et formant deux cordons latéraux qui peuvent s'étendre le long ou croiser lesdites parties latérales (6) et être reliés ensemble de manière à tenir étroitement ladite partie intermédiaire (5) contre le bord inférieur de l'apophyse épineuse de ladite première vertèbre (L5) ;
dans laquelle l'implant (3) comprend une partie centrale et un revêtement extérieur ;
**caractérisée en ce que** ;
la partie centrale est une structure composite, comprenant un élément intérieur fait d'un matériau non compressible et un élément extérieur recouvrant l'élément intérieur, en formant une couche plus épaisse que l'élément intérieur dans un matériau viscoélastique malléable absorbant les chocs.

2. Prothèse vertébrale (2) selon la revendication 1, comprenant en outre un implant interépineux (1) destiné à être placé au niveau de l'articulation vertébrale située au-dessus de ladite première vertèbre (L5), à savoir entre ladite première vertèbre (L5) et une troisième vertèbre (L4), et dans laquelle l'implant (3) est distinct et séparé dudit implant interépineux (1).

3. Prothèse vertébrale (2) selon la revendication 1, comprenant en outre une partie interépineuse supérieure (1), absorbant les chocs, destinée à être placée sur l'articulation vertébrale entre ladite première vertèbre (L5) et une troisième vertèbre (L4) située au-dessus de cette première vertèbre (L5) ; dans laquelle l'implant (3) fait partie intégrante de la partie interépineuse supérieure (1), et l'implant (3) définit, avec cette partie interépineuse supérieure (1), une ouverture permettant l'insertion de l'implant (3) sur l'apophyse épineuse de ladite première vertèbre (L5).

4. Prothèse vertébrale (2) selon l'une quelconque des revendications 1 à 3, **caractérisée en ce que** le revêtement extérieur est fait d'un matériau textile tissé ou de tout autre matériau ayant un maillage approprié.

5. Prothèse vertébrale (2) selon l'une quelconque des revendications 1 à 4, **caractérisée en ce que** ladite partie intermédiaire (5) de l'implant (3) comprend au moins une cavité ou encoche destinée à permettre l'application précise de l'implant contre le bord inférieur de l'apophyse épineuse de ladite première vertèbre (L5) et/ou contre ladite seconde vertèbre (S1), en permettant ainsi la stabilisation de cet implant par rapport à cette ou ces apophyse(s) épineuse(s).

6. Prothèse vertébrale (2) selon l'une quelconque des revendications 2 à 4, **caractérisée en ce que** l'extrémité libre d'au moins l'une des parties latérales (6) de l'implant (3) comprend une cavité ou encoche permettant à l'implant (3) de s'accoupler à un implant interépineux (1) destiné à être placé entre lesdites première et troisième vertèbres (L4, L5).
